Europäisches Patentamt

European Patent Office

Office européen des brevets

(19)

(11) Publication number: **0 250 134 B1**

## (12) EUROPEAN PATENT SPECIFICATION

(45) Date of publication of patent specification: **11.11.92**

(51) Int. Cl.⁵: **A61K 31/35**, A61K 35/72, A23K 1/17

(21) Application number: **87305000.9**

(22) Date of filing: **05.06.87**

(54) **Method for combating swine dysentery.**

(30) Priority: **06.06.86 US 872663**

(43) Date of publication of application:
**23.12.87 Bulletin 87/52**

(45) Publication of the grant of the patent:
**11.11.92 Bulletin 92/46**

(84) Designated Contracting States:
**AT BE CH DE ES FR GB GR IT LI LU NL SE**

(56) References cited:
**EP-A- 0 099 420**
**GB-A- 2 025 226**
**GB-A- 2 055 094**
**US-A- 4 478 935**

**Diseases of Swine, 6th ed., 1986, pp 494-507, pp148, 149, pp219, 220**

(73) Proprietor: **IMCERA Group Inc.**
**2315 Sanders Road**
**Northbrook, IL 60062(US)**

(72) Inventor: **Williams, Robert D.**
**Rural Route 25**
**Terre Haute Indiana 47802(US)**
Inventor: **Power, Carl**
**7377, Whip-poor-will Drive**
**Terre Haute Indiana 47802(US)**

(74) Representative: **Holmes, Michael John et al**
**Frank B. Dehn & Co. European Patent Attorneys Imperial House 15-19 Kingsway**
**London, WC2B 6UZ,(GB)**

EP 0 250 134 B1

**Description**

This invention relates generally to the prevention and control of swine dysentery and, in particular, to the use of lysocellin to control and prevent swine dysentery.

Swine dysentery is an enteric disease characterized pathologically by inflammation of the colon and cecal mucosa and clinically by dehydration, loss of body weight, muco-hemorrhagic diarrhea, and lesions of the large intestine. Swine dysentery is one of the major problems for swine producers and is estimated to cost millions of dollars yearly in lost productivity. It is generally accepted that the spirochete, Treponema hyodysenteriae, is the prime cause of swine dysentery.

Many chemotherapeutic agents and antibiotics have been evaluated for their prophylactic or therapeutic effects against swine dysentery. Arsenic compounds such as arsanilic acid were among the first compounds found to be effective against swine dysentery. However, swine treated with effective levels of arsenic compounds developed signs of arsenic poisoning making their use dangerous to the swine. Sulfonamides and nitrofurans have been used to control dysentery but have not been very effective. Antibiotics such as bacitracin, streptomycin, penicillin, chlortetracycline, oxytetracycline and tylosin have been used with limited effectiveness often requiring very large dosages to control dysentery. These antibiotics, however, generally are toxic at high dosages and are not palatable. This makes it difficult to administer them in feed and water at high concentrations. A method, therefore, is needed for effectively controlling and preventing swine dysentery which will permit a broad range of dosage levels without toxicity or swine rejections because of the bad taste.

The following methods for controlling swine dysentery are disclosed in the prior art. U.S. Patent No. 4,086,345 to Garzia and Williams discloses the use of substituted quinoxalines to combat swine dysentery. U.S. Patent No. 4,027,034 to Messersmith discloses a method for the prevention of swine dysentery by the oral administration of the antibiotic monensin and several other polyether antibiotics to swine susceptible to the disease. U.S. Patent No. 4,436,734 to Ose discloses the use of the antibiotic avilamycin for the treatment and prevention of swine dysentery. Salinomycin is reported to be effective in the treatment of swine dysentery in U.S. Patent No. 4,291,053.

A method for the production of the antibiotic lysocellin was disclosed in U.S. Patent No. 4,033,823. The method involves the cultivation of a strain of Streptomyces longwoodenis which is on deposit at the American Type Culture Collection under the designation ATCC 29251.

A method for the production of the antibiotic carriomycin is disclosed in U.S. Patent No. 4,069,316. The method involves the cultivation of the T-42082 strain of Streptomyces hygroscopicus which is on deposit at the American Type Culture Collection under the designation ATCC 31080.

It is, therefore, an object of the present invention to provide a method for the prevention and control of swine dysentery.

It is another object of the present invention to provide a method for controlling swine dysentery which uses an antibiotic that is non-toxic and palatable to swine in large doses.

It is another object of the present invention to provide a method for controlling swine dysentery which uses an antibiotic which can be administered in large doses in swine food and water.

It is a further object of the present invention to provide a non-toxic and palatable swine feed composition containing an antibiotic suitable for controlling swine dysentery.

These and other objects are achieved by administering orally an effective amount of an antibiotic compound selected from lysocellin or its pharmaceutically-acceptable salts to swine suffering from or susceptible to swine dysentery. As used herein, the term lysocellin refers to the compounds and its pharmaceutically-acceptable salts and esters, including but not limited to the Zn, Cu, Na, Mn, Ni and Co salts and the methyl, ethyl propyl, butyl, and other esters.

Accordingly, there is provided use of an antibiotic selected from lysocellin, and pharmaceutically acceptable salts and esters thereof for the preparation of a composition comprising an effective amount of said antibiotic and an edible solid or liquid carrier material for the treatment or prevention of Swine dysentery caused by Treponema hyodysenteriae.

Other objects, advantages, and novel features of the present invention will become apparent from the following detailed description of the invention.

In accordance with the method of the present invention, swine that may contract swine dysentery are treated with lysocellin at a dosage from 1-5 milligrams per kilogram of body weight per day to prevent the disease. The antibiotic can conveniently be administered by mixing the antibiotic with the swine feed in an amount from 19.69-196.85 grams per tonne (t) of feed (20-200 grams/T). Alternatively, the antibiotic can be administered to the swine in drinking water by adding from 0.0176-0.044 grams of antibiotic per I of water (0.04-0.2 g/gallon).

2

Swine that have contracted swine dysentery can be treated using the above antibiotics at a dosage from 5-25 milligrams per kilogram body weight per day to control the disease. The antibiotic can conveniently be administered by mixing the antibiotics with the swine feed in an amount from 98.43-492.13 grams per tonne (t) (100-500 grams/T). Alternatively, the antibiotics can be administered to the swine in drinking water by adding from 0.044-0.22 grams antibiotic per litre of water (0.2-1.0 grams/gallon).

A swine feed for oral administration of the antibiotics of the present invention can easily be prepared by admixing the antibiotics alone or as a premix with a conventional nutritional swine feed to produce a homogeneous feed product. A typical swine feed composition may be prepared containing the usual nutritionally-balanced quantities of carbohydrates, proteins, vitamins and minerals, together with the antibiotics in accordance with the present invention. Some of the usual dietary elements included in swine feed compositions are grains, such as ground grain and grain byproducts, animal protein substances, such as those found in fish meal and meat scraps, vegetable proteins, like soybean oil meal or peanut oil meal; vitamins and vitamin-containing materials, e.g., vitamin A and D mixtures, riboflavin supplements and other vitamin B complex members; and bone meal and limestone to provide minerals.

Additionally, the swine feed can be prepared by sprinkling the antibiotic on the swine feed in the form of a powder or as pellets. Any other suitable method can be used to administer the antibiotic orally and it is not intended that the invention be limited to any particular mode of administration.

Lysocellin and its salts and esters are surprisingly effective in controlling swine dysentery since they can be administered to the swine in large doses. The compounds can be safely administered in the swine feed at concentrations up to 25 mg of antibiotic per kilogram of body weight per day. The compounds are typically administered by placing them in the swine feed at a level of up to 196.85 grams of antibiotic (/t (200 grams/T) or in the swine drinking water at a level up to 0.044 grams of antibiotic per litre of water (0.2 g/gallon).

At these levels, the swine neither becomes sick because of the large dose of antibiotic nor rejects the feed because of the bad taste that accompanies other similar antibiotics. The swine are also able to maintain weight gain even in the presence of the disease.

Lysocellin is an acid and reacts with organic and inorganic bases to form salts. Examples of mineral bases forming pharmaceutically acceptable cationic salts with lysocellin include the alkaline metal hydroxides such as lithium hydroxide, sodium hydroxide and potassium hydroxide, the carbonates and the bicarbonates of alkaline metals such as lithium carbonate and sodium bicarbonate; hydroxides and carbonates of alkaline earth metals such as calcium hydroxide, magnesium carbonates; and similar mineral bases.

As examples of organic bases forming pharmaceutically acceptable salts with lysocellin are lower alkyl amines, primary, secondary and tertiary hydroxy lower alkyl amines such as ethylamine, isopropylamine, diethylamine, methyl-n-butylamine, ethanolamine and diethanolamine. The ammonium salts of lysocellin are prepared with ammonia or ammonium hydroxide.

The pharmaceutically acceptable esters of the acid group of lysocellin are easily obtained. For example, the alkyl esters such as methyl, isopropyl and butyl esters, cycloalkyl esters such as cyclopropyl and cyclohexyl esters, and aryl esters such as phenyl ester can be obtained by reaction of the acid with a diazo derivative of the substituent to be added.

It is equally possible to obtain esters by acylation of one or more hydroxyl groups of these antibiotics. For example, esters are obtained by acylation with groups such as formyl, acetyl hexanoyl, and benzoyl groups or by a reaction with an anhydride of the acyl group to be added. The salts and esters of lysocellin as described above are all useful in the process of this invention.

The invention having been generally described, the following examples are given as particular embodiments of the invention and to demonstrate the practice and advantages thereof. It is understood that the examples are given by way of illustration and are not intended to limit the specification or the claims to follow in any manner. As used herein, the terms swine and pig are synonomous.

In vitro Activity

The following test procedure was used to evaluate the anti-Treponema hyodysenteriae activity of the compounds in the present invention. This test procedure establishes the lowest concentration of the compounds that will inhibit the growth on cultures of Treponema hyodysenteriae in an in vitro Streak Plate Test Method.

Procedure: Stock solutions containing 10,000, 1,000, 100, 10, 1.0, and 0.1, micrograms per milliliters of the compound to be tested were prepared. The final dilutions to establish the test concentrations in the media were made by placing the correct volume of stock solution in a petri dish, adding the media

EP 0 250 134 B1

(trypticase soy blood agar), and mixing before the media solidified. After the media solidified, the test cultures of Treponema hyodysenteriae were streaked onto the surface of the agar. Petri dishes containing media without test samples were also set up as culture controls and are inoculated in the same manner. All inoculated plates were incubated at 39°C in anaerobic jars for 48 hrs. Inhibition endpoints were then determined by observing the test plates for growth or no growth of the test cultures at the various concentrations of the compounds. If the culture growth occured at one concentration but not at the next higher concentration, the inhibitory endpoint or MIC (Minimum Inhibitory Concentration) was considered the higher concentration. The results are summarized in Table 1.

Referring to Table 1, the culture numbers refer to field isolate strains of Treponema hyodysenteriae. These field isolates were isolated in Illinois, Iowa, and Nebraska. The numbers giving the results in the Table are read as follows: the first number for each isolate and compound tested corresponds to the concentration in micrograms per milliliter where growth occurred and the second number corresponds to the concentration in micrograms per milliliter where no growth occurred. Refering to the results in the Table, lysocellin and its cationic salts exhibited significant activity against Treponema hyodysenteriae at concentrations less than 0.01 micrograms per milliliter. Carriomycin and lysocellin showed comparable activity when compared to the controls used in this experiment.

In vivo Activity

One hundred twenty (120) cross-bred barrows and gilts were used in a randomized complete blocks design to evaluate the effect of lysocellin on Treponema hyodysenteriae induced swine dysentery. The swine were divided into 4 groups: (1) non-infected, non-medicated controls, (2) infected non-medicated controls, (3) infected, medicated at 98.43 grams lysocellin/t (100 g/T), and (4) infected, medicated at 196.85 grams lysocellin/t (200 g/T). All were observed through a 51-day trial period. Swine in groups (2)-(4) were challenged with Treponema hyodysenteriae on study day 22 and the post-challenge period data were analyzed for significant differences. A description of the experimental design is summarized as follows:

| Treatment No. | g/t | Challenged | Pigs/pen | Replicates | Pigs/Treatment |
|---|---|---|---|---|---|
| 1-nonmedicated, nonchallenged | 0 | No | 5 | 6 | 30 |
| 2-nonmedicated, challenged | 0 | Yes | 5 | 6 | 30 |
| 3-medicated, challenged | 98,43 | Yes | 5 | 6 | 30 |
| 4-medicated, challenged | 196,85 | Yes | 5 | 6 | 30 |

Comparisons were made on treatment 1 vs. treatments 3 and 4, treatment 2 vs. treatment 3 and 4, and treatment 3 vs. treatment 4. The following observations were made based upon data analyzed: postchallenge performance of swine with respect to daily gain and daily feed gain, number of pigs per pen expressing clinical signs of dysentery during the postchallenge period, number of pig days per pen with clinical signs of dysentery during the postchallenge period, and number of pig days per pen with specified fecal score. The results of the study are summarized in Table 2, Table 3, Table 4 and Table 5.

Referring to Table 2, cumulative average daily gain of noninfected control pigs (treatment 1) was significantly greater (P<.05) at 14, 21 and 28 days than the average of the daily gains of pigs receiving 98.43 or 196.85 g lysocellin/t (1000-200g/T) (treatments 3 and 4 respectively). Cumulative feed intake of the non-infected control pigs was also greater (P<.05) than that of medicated pigs over these same three periods. There was no significant difference (P<.05) in feed:gain between noninfected control pigs and pigs receiving lysocellin.

Infected pigs provided with lysocellin performed significantly (P<.05) better than infected, non-medicated control pigs (treatment 2) receiving no lysocellin. In each period (7,14,21 and 28 days), cumulative average daily gain for the pigs receiving lysocellin was improved (P<.05) over that of nonmedicated pigs. Although pigs receiving lysocellin consumed more (P<.05) feed than infected control pigs, feed efficiency of the pigs receiving lysocellin was superior (P<.05).

Significant differences between performance of pigs receiving the two levels of lysocellin were observed for cumulative daily gain at 14, 21 and 28 days and for cumulative feed intake and feed efficiency at 21 days. Pigs receiving 196.85 g lysocellin/t (200 g/T) had significantly greater gain (P<.05) and feed intake (P<.05) than those receiving 98.43 g lysocellin/t (100 g/T) for the periods noted above. Feed efficiency was improved (P<.05) at 21 days due to inclusion of 196.85 vs 98.43 g lysocellin/t (200 vs 100 g/T) in the diet.

Referring to table 3 and table 4, clinical signs of dysentery observed during the postchallenge period are presented as treatment means of: number of pigs per pen expressing the sign and number of pig days

4

the sign was observed within a pen. The value "pig days" was based on 28 days/pig surviving to the end of the trial or number of days a pig survived during the postchallenge period (for example, 5 pigs/pen on trial for 28 days equals 140 pig days). Removal of pigs from a pen during the trial was taken into account in these calculations.

Noninfected control pigs showed no sign of weakness, depression, dehydration, reddening of the skin, anorexia or bloody feces. One pig in this group appeared lethargic for 1 day and another appeared gaunt and had a rough hair coat at 3 and 9 days, respectively. These signs were judged not to be related to infection with Treponema hyodsenteriae. Infected pigs receiving either level of lysocellin appeared significantly more (P<.05) lethargic, gaunt, weak, depressed and dehydrated and showed more signs of rough hair coat, reddening of the skin and bloody feces than did the noninfected control pigs on a number of pigs basis. This was also true for number of pig days, although there were no differences (P<.05) in weakness or reddening of the skin. No pigs receiving lysocellin appeared anorexic.

Considering number of pigs and pig days, infected non-medicated control pigs were significantly more (P<.05) lethargic, gaunt, weak, depressed and anorexic and showed more signs of bloody feces than did the pigs receiving lysocellin. A greater (P<.05) number of infected control pigs had rough hair coats. Infected non-medicated control pigs also appeared more (P<.05) dehydrated for a greater number of pig days.

Pigs receiving 98.43 g lysocellin/t (100 g/T), on a number of pigs or number of pig day basis, appeared significantly more (P<.05) lethargic, gaunt, depressed and dehydrated and showed more signs of bloody feces than did those receiving 196.85 g lysocellin/t (200 g/T).

Referring to table 5, fecal condition during the postchallenge period of the trial is expressed as the number of pen pig days with a specified fecal condition. Noninfected control pigs exhibited no sign of diarrhea during the postchallenge period. Soft feces were observed 5% of the time. This is considered normal and due to individual pig and day-to-day variation. Challenging the pigs with T. hyodysenteriae decreased the number of pig days the pigs had normal feces while increasing the number of days with soft feces, watery diarrhea or bloody diarrhea. Pigs receiving lysocellin had significantly fewer (P<.05) days with normal feces and more (P<.05) days with soft feces, water diarrhea or bloody diarrhea than noninfected control pigs. Infected non-medicated control pigs had significantly fewer (P<.05) days of normal feces and more (P<.05) days of water or bloody diarrhea than pigs receiving lysocellin. Supplying pigs with 196.85 g lysocellin/t (200 g/T) rather than 98.43 g/T increased (PM.05) the number of days with normal feces and decreased (P<.05) the incidence of soft, watery or bloody feces to a level closer to that observed in non-infected control pigs.

Feeding lysocellin, therefore, prevented mortality and resulted in fewer clinical signs in overall pig performance similar to that of noninfected, nonmedicated pigs.

Obviously many modifications and variations of the present invention are possible in light of the above teachings. It is therefore to be understood that within the scope of the appended claims the invention may be practiced otherwise than as specifically described.

EP 0 250 134 B1

TABLE 1
Inhibition Endpoints in µgs/ml

| | Culture Nos. | | | | | | | | |
| | 8080 | 8098 | 8087 | 8072 | 8064 | 8076 | 8125 | B-140 | B-100 |
|---|---|---|---|---|---|---|---|---|---|
| Lysocellin Free Acid | .1-.5 | .05-.1 | .05-.1 | .01-.05 | .1-.5 | <.01 | .01-.05 | .01-.1 | .01-.05 |
| Lysocellin Na | .1-.5 | .1-.5 | .1-.5 | .1-.5 | .1-.5 | .1-.5 | .1-.5 | .1-.5 | .1-.5 |
| Na Zn Lysocellin | .5-1.0 | .1-.5 | 1.0-5.0 | 1.0-5.0 | 1.0-5.0 | 1.0-5.0 | 1.0-5.0 | 1.0-5.0 | 1.0-5.0 |
| Lasalocid Na | .5-1.0 | 1.0-5.0 | 1.0-5.0 | 1.0-5.0 | 1.0-5.0 | 1.0-5.0 | 1.0-5.0 | 1.0-5.0 | .5-1.0 |
| Lincomycin HCL | 10-50 | 10-50 | 10-50 | 10-50 | 10-50 | 10-50 | 10-50 | .05-.1 | 1.0-5.0 |
| Salinomycin Na | .01-.05 | .01-.05 | .01-.05 | .01-.05 | .01-.05 | .01-.05 | .01-.05 | .01-.05 | .01-.05 |
| Carriomycin Na | .01-.05 | <.01 | .01-.05 | <.01 | .01-.05 | <.01 | <.01 | <.01 / <.05 | <.01 / <.05 |
| Monensin Na | .05-.1 | .01-.05 | .01-.05 | .01-.05 | .05-.1 | .01-.05 | .01-.05 | .05-.1 / <.05 | .05-.1 / .05-.1 |

TABLE 1  cont'd

| | 8080 | 8098 | 8087 | Culture Nos. 8072 | 8064 | 8076 | 8125 | B-140 | B-100 |
|---|---|---|---|---|---|---|---|---|---|
| Mn Lysocellin | .1-.5 | .1-.5 | .1-.5 | .1-.5 | .1-.5 | .1-.5 | .1-.5 | .1-.5 | .1-.5 |
| Zn Lysocellin | .1-.5 | .1-.5 | .05-.1 | .1-.5 | .1-.5 | .1-.5 | .05-.1 | .05-.1 | .05-.1 |
| Cu Lysocellin | .1-.5 | .05-.1 | .1-.5 | .05-.1 | .1-.5 | .05-.1 | .1-.5 | .1-.5 | .1-.5 |
| Ni Lysocellin | .1-.5 | .05-.1 | .05-.1 | <.01 | .05-.1 | .05-.1 | <.01 | <.01 | <.01 |
| Co Lysocellin | .05-.1 | .05-.1 | .05-.1 | <.01 | .05-.1 | .05-.1 | <.01 | <.01 | <.01 |

EP 0 250 134 B1

TABLE 2
Postchallenge Performance of Swine

| Item | Period, days | Treatment[a] | | | | SEM[b] |
| | | 1 | 2 | 3 | 4 | |
|---|---|---|---|---|---|---|
| Daily gain, kg/d[d] | 0-7 | 0.42 | 0.11 | 0.35 | 0.41 | 0.03 |
| Daily gain, kg/d[cde] | 0-14 | 0.40 | 0.09 | 0.22 | 0.35 | 0.03 |
| Daily gain, kg/d[cde] | 0-21 | 0.48 | 0.13 | 0.28 | 0.42 | 0.03 |
| Daily gain, kg/d[cde] | 0-28 | 0.49 | 0.19 | 0.33 | 0.45 | 0.03 |
| Daily feed, kg/d | 0-7 | 1.03 | 0.85 | 1.06 | 0.94 | 0.08 |
| Daily feed, kg/d[cd] | 0-14 | 1.17 | 0.69 | 0.90 | 1.03 | 0.06 |
| Daily feed, kg/d[cde] | 0-21 | 1.28 | 0.67 | 0.89 | 1.07 | 0.06 |
| Daily feed, kg/d[cd] | 0-28 | 1.37 | 0.73 | 0.99 | 1.15 | 0.06 |
| Feed/Gain[d] | 0-7 | 2.48 | 9.39 | 3.21 | 2.30 | 1.35 |
| Feed/Gain[d] | 0-14 | 2.96 | 8.27 | 4.61 | 2.99 | 0.98 |
| Feed/Gain[de] | 0-21 | 2.68 | 4.93 | 3.38 | 2.55 | 0.28 |
| Feed/Gain[d] | 0-28 | 2.78 | 6.50 | 3.01 | 2.55 | 1.32 |

[a] 1 = noninfected, control; 2= infected, control; 3 = 98.43 g/t (100 g/T) lysocellin; 4 = 196.85 g/t (200 g/T) lysocellin.

[b] Standard error of the mean.

[c] 1 vs 3,4 significantly different (P<.05).

[d] 2 vs 3,4 significantly different (P<.05).

[e] 3 vs 4 significantly different (P<0.05).

TABLE 3

Number of Pigs per Pen Expressing Clinical Signs
of Dysentery During the Postchallenge Period[a]

| Item | Treatment[b] | | | |
|---|---|---|---|---|
| | 1 | 2 | 3 | 4 |
| Lethargic[cde] | 0.13 (-0.15,0.49) | 4.16 (3.32,5.08) | 2.12 (1.50,2.82) | 0.43 (0.08,0.87) |
| Gauntness[cde] | 0.13 (-0.16,0.51) | 4.66 (3.74,5.66) | 3.98 (3.13,4.92) | 1.96 (1.35,2.67) |
| Weak[cd] | 0.00 (-0.26,0.35) | 2.95 (2.20,3.79) | 1.93 (1.31,2.64) | 1.31 (0.78,1.93) |
| Depressed[cde] | 0.00 (-0.26,0.35) | 2.40 (1.71,3.17) | 1.78 (1.18,2.47) | 0.27 (-0.06,0.70) |
| Dehydrated[ce] | 0.00 (-0.33,0.49) | 0.87 (0.28,1.63) | 1.08 (0.44,1.89) | 0.23 (-0.18,0.80) |
| Anorexia[d] | 0.00 (-0.27,0.37) | 0.68 (0.23,1.22) | 0.00 (-0.27,0.37) | 0.00 (-0.27,0.37) |
| Rough hair coat[cd] | 0.13 (-0.21,0.60) | 2.95 (2.07,3.97) | 1.93 (1.20,2.79) | 1.31 (0.68,2.06) |
| Reddened skin[c] | 0.00 (-0.34,0.54) | 0.61 (0.05,1.36) | 0.87 (0.24,1.69) | 0.55 (0.01,1.28) |
| Bloody feces[cde] | 0.00 (-0.28,0.40) | 4.66 (3.62,5.81) | 2.73 (1.93,3.66) | 1.20 (0.63,1.89) |
| Any clinical signs[cde] | 0.27 (-0.11,0.79) | 5.00 (3.87,6.26) | 4.49 (3.42,5.69) | 2.72 (1.87,3.70) |

[a]Values in parentheses are lower and higher 95% confidence limits of the mean.

[b]1 = noninfected, control; 2 = infected, control; 3 = 98.43 g/t (100 g/T) lysocellin;
4 = 196.85 g/t (200 g/T) lysocellin.

[c]1 vs 3,4 significantly different (P<.05).

[d]2 vs 3,4 significantly different (P<.05).

[e]3 vs 4 significantly different (P<.05).

EP 0 250 134 B1

TABLE 4
Number of Pig Days per Pen with Clinical Signs
of Dysentery During the Postchallenge Period[a]

| Item | Treatment[b] | | | |
|---|---|---|---|---|
| | 1 | 2 | 3 | 4 |
| Lethargic[cde] | 0.13 (-0.50,1.93) | 14.80 (9.39,21.38) | 9.08 (4.93,14.42) | 1.82 (0.07,4.74) |
| Gauntness[cde] | 0.31 (-0.50,2.82) | 39.24 (28.48,51.69) | 30.35 (20.97,41.42) | 11.17 (5.73,18.30) |
| Weak[d] | 0.00 (-0.41,0.74) | 6.97 (4.91,9.36) | 0.87 (0.08,1.99) | 0.13 (-0.35,0.94) |
| Depressed[cde] | 0.00 (-0.40,0.72) | 4.58 (2.95,6.54) | 3.14 (1.78,4.81) | 0.38 (-0.21,1.29) |
| Dehydrated[cde] | 0.00 (-0.41,0.75) | 3.35 (1.91,5.13) | 2.69 (1.40,4.33) | 0.23 (-0.30,1.09) |
| Anorexia[d] | 0.00 (-0.30,0.43) | 1.04 (0.46,1.75) | 0.00 (-0.30,0.43) | 0.00 (-0.30,0.43) |
| Rough hair coat[c] | 0.72 (-0.35,6.21) | 27.24 (13.78,45.12) | 18.07 (7.46,33.11) | 9.55 (2.33,21.19) |
| Reddened skin | 0.00 (-0.50,1.32) | 1.28 (-0.02,3.39) | 2.21 (0.51,4.72) | 1.29 (-0.02,3.41) |
| Bloody feces[cde] | 0.00 (-0.50,1.60) | 16.05 (10.56,22.64) | 9.98 (5.72,15.34) | 3.02 (0.78,6.35) |

[a] Values in parentheses are lower and higher 95% confidence limits of the mean.
[b] 1 = noninfected, control; 2 = infected, control; 3 = 98.43 g/t (100 g/T) lysocellin;
[c] 1 vs 3,4 significantly different (P<.05).          4 = 196.85 g/t (200 g/T) lysocellin.
[d] 2 vs 3,4 significantly different (P<.05).
[e] 3 vs 4 significantly different (P<.05).

EP 0 250 134 B1

TABLE 5
Number of Pig Days Per Pen with Specified Fecal Score[a]

| Item | Treatment[b] | | | |
|---|---|---|---|---|
| | 1 | 2 | 3 | 4 |
| Normal[cde] | 132.90 (122.11,145.24) | 43.70 (37.03,50.92) | 79.51 (70.44,89.12) | 122.57 (111.25,134.43) |
| Soft[ce] | 5.24 (1.44,11.07) | 23.06 (14.31,33.82) | 26.76 (17.28,38.26) | 10.01 (4.50,17.53) |
| Watery[cde] | 0.00 (-0.50,1.37) | 15.54 (10.69,21.25) | 9.27 (5.59,13.83) | 2.72 (0.79,5.52) |
| Bloody[cde] | 0.00 (-0.50,1.74) | 17.68 (11.58,25.02) | 9.98 (5.50,15.70) | 3.02 (0.68,6.59) |

[a] Values in parentheses are lower and higher 95% confidence limits of the mean.

[b] 1 = noninfected, control; 2 = infected, control; 3 = 98.43 g/t (100 g/T) lysocellin; 4 = 196.85 g/t (200 g/T) lysocellin.

[c] 1 vs 3,4 significantly different (P<.05).

[d] 2 vs 3,4 significantly different (P<.05).

[e] 3 vs 4 significantly different (P<.05).

**Claims**

1. Use of an antibiotic selected from lysocellin, and pharmaceutically acceptable salts and esters thereof for the preparation of a composition comprising an effective amount of said antibiotic and an edible

11

solid or liquid carrier material for the treatment or prevention of swine dysentery caused by treponema hyodysenteriae.

2. Use as claimed in claim 1, wherein said composition is a non-toxic and palatable swine feed composition comprising a nutritional swine feed and an effective amount of said antibiotic.

3. Use as claimed in claim 1, wherein an aqueous solution containing said antibiotic is prepared.

4. Use as claimed in claim 1 or claim 2 for the preparation of a feed composition containing up to 196.85 grams of said antibiotic per tonne of feed (200 grams per ton) for use in the prevention of swine dysentery caused by treponema hyodysenteriae.

5. Use as claimed in claim 4 for the preparation of a composition containing 19.69 to 196.85 grams of said antibiotic per tonne of feed (20 to 200 grams per ton).

6. Use as claimed in claim 1 or claim 2 for the preparation of a feed composition containing 98.43 to 492.13 grams of said antibiotic per tonne of feed (100 to 500 grams per ton) for use in the treatment of swine dysentery caused by treponema hyodysenteriae.

7. Use as claimed in claim 3 for the preparation of an aqueous solution containing up to 0.044 grams of said antibiotic per litre of water (0.2 grams per gallon) for use in the prevention of swine dysentery caused by treponema hyodysenteriae.

8. Use as claimed in claim 7 for the preparation of an aqueous solution containing 0.0176 to 0.044 grams of said antibiotic per litre of water (0.04 to 0.2 grams per gallon) for use in the prevention of swine dysentery caused by treponema hyodysenteriae.

9. Use as claimed in claim 3 for the preparation of an aqueous solution containing 0.044 to 0.22 grams of said antibiotic per litre of water (0.2 to 1.0 grams per gallon) for use in the treatment of swine dysentery caused by treponema hyodysenteriae.

**Patentansprüche**

1. Verwendung eines Antibiotikums, ausgewählt unter Lysocellin und den pharmazeutisch verträglichen Salzen und Estern davon zur Herstellung einer Zusammensetzung zur Behandlung oder Prävention von Schweinedysenterie, welche durch Treponema hyodysenteriae verursacht ist, wobei die Zusammensetzung eine wirksame Menge des Antibiotikums und ein genießbares festes oder flüssiges Trägermaterial umfaßt.

2. Verwendung nach Anspruch 1, wobei die Zusammensetzung eine nicht-toxische und angenehm schmeckende Schweinefutterzusammensetzung ist, welche ein Schweinefutter und eine wirksame Menge des Antiobiotikums umfaßt.

3. Verwendung nach Anspruch 1, wobei man eine das Antibiotikum enthaltende wäßrige Lösung herstellt.

4. Verwendung nach Anspruch 1 oder 2 zur Herstellung einer Futterzusammensetzung zur Prävention von Schweinedysenterie, welche durch Treponema hyodysenteriae verursacht ist, wobei die Zusammensetzung bis zu 196,85 g des Antibiotikums pro Tonne Futter (200 g/Tonne) enthält.

5. Verwendung nach Anspruch 4 zur Herstellung einer Zusammensetzung, die 19,69 bis 196,85 g des Antibiotikums pro Tonne Futter (20 - 200 g/Tonne) enthält.

6. Verwendung nach Anspruch 1 oder 2 zur Herstellung einer Futterzusammensetzung zur Behandlung von Schweinedysenterie, welche durch Treponema hyodysenteriae verursacht ist, wobei die Zusammensetzung 98,43 bis 492,13 g des Antibiotikums pro Tonne Futter (100 - 500 g/Tonne) enthält.

7. Verwendung nach Anspruch 3 zur Herstellung einer wäßrigen Lösung zur Prävention von Schweinedysenterie, welche durch Treponema hyodysenteriae verursacht ist, wobei die Lösung bis zu 0,044 g des Antibiotikums pro Liter Wasser (0,2 g/Gallone) enthält.

8. Verwendung nach Anspruch 7 zur Herstellung einer wäßrigen Lösung zur Prävention von Schweinedysenterie, welche durch Treponema hyodysenteriae verursacht ist, wobei die Lösung 0,0176 bis 0,044 g des Antibiotikums pro Liter Wasser (0,04 - 0,2 g/Gallone) enthält.

9. Verwendung nach Anspruch 3 zur Herstellung einer wäßrigen Lösung zur Behandlung von Schweinedysenterie, welche durch Treponema hyodysenteriae verursacht ist, wobei die Lösung 0,044 bis 0,22 g des Antibiotikums pro Liter Wasser (0,2 - 1,0 g/Gallone) enthält.

**Revendications**

1. Utilisation d'un antibiotique choisi parmi la lysocelline et les sels et esters pharmaceutiquement acceptables de celle-ci en vue de la préparation d'une composition comprenant une proportion efficace de l'antibiotique précité et une matière servant de véhicule ou d'excipient, liquide ou solide, comestible, pour le traitement ou la prévention de la dysenterie porcine provoquée par treponema hyodosenteriae.

2. Utilisation suivant la revendication 1, caractérisée en ce que la composition est une composition alimentaire destinée aux porcs, atoxique et sapide, comprenant un aliment de nutrition du porc et une quantité efficace de l'antibiotique précité.

3. Utilisation suivant la revendication 1, caractérisé en ce que l'on prépare une solution aqueuse contenant l'antibiotique précité.

4. Utilisation suivant la revendication 1 ou la revendication 2, en vue de la préparation d'une composition alimentaire contenant jusqu'à 196,85 grammes de l'antibiotique précité par tonne d'aliments (200 grammes par tonne) que l'on utilise pour la prévention de la dysenterie porcine provoquée par treponema hyodysenteriae.

5. Utilisation suivant la revendication 4, en vue de la préparation d'une composition contenant 19,69 à 196,85 grammes de l'antibiotique précité par tonne d'aliments (20 à 200 grammes par tonne).

6. Utilisation suivant la revendication 1 et la revendication 2, en vue de la préparation d'une composition alimentaire contenant 98,43 à 492,13 grammes de l'antibiotique précité par tonne d'aliments (100 à 500 grammes par tonne) en vue du traitement de la dysenterie porcine provoquée par treponema hyodysenteriae.

7. Utilisation suivant la revendication 3, en vue de la préparation d'une solution aqueuse contenant jusqu'à 0,044 gramme de l'antibiotique précité par litre d'eau (0,2 grammes par gallon) en vue de l'utilisation pour la prévention de la dysenterie porcine provoquée par treponema hyodysenteriae.

8. Utilisation suivant la revendication 7, en vue de la préparation d'une solution aqueuse contenant 0,0176 à 0,044 gramme de l'antibiotique précité par litre d'eau (0,04 à 0,2 gramme par gallon) en vue de l'utilisation pour la prévention de la dysenterie porcine provoquée par treponema hyodysenteriae.

9. Utilisation suivant la revendication 3, en vue de la préparation d'une solution aqueuse contenant 0,044 à 0,22 gramme de l'antibiotique précité par litre d'eau (0,2 à 1,0 gramme par gallon) en vue de l'utilisation pour le traitement de la dysenterie porcine provoquée par treponema hyodysenteriae.